# FASCICULE DE BREVET EUROPEEN

(11) **EP 0 642 528 B1**
(45) Date de publication et mention de la délivrance du brevet: **15.01.1997**
(21) Numéro de dépôt: 93910147.3
(22) Date de dépôt: 24.05.1993
(51) Int. Cl.: C07H 19/10, C07H 19/20, C07F 9/6561, A61K 31/70

(54) **COMPOSES BIOLOGIQUEMENT ACTIFS DE TYPE PHOSPHOTRIESTERS**
PHOSPHOTRIESTER-TYP BIOLOGISCH-AKTIVER VERBINDUNGEN
PHOSPHOTRIESTER-TYPE BIOLOGICALLY ACTIVE COMPOUNDS

(30) Priorité: 25.05.1992 FR 9206383; 07.04.1993 FR 9304117
(43) Date de publication de la demande: 15.03.1995
(73) Titulaire: CENTRE NATIONAL DE LA RECHERCHE SCIENTIFIQUE, 75007 Paris (FR)
(72) Inventeur: GOSSELIN, Gilles, Résidence "Parc-des-Arceaux", F-34000 Montpellier (FR); IMBACH, Jean-Louis, Lab. de Chimie Bio Organique, F-34095 Montpellier (FR)
(74) Mandataire: Martin, Jean-Jacques
(86) Numéro de dépôt international: FR9300498
(87) Numéro de publication internationale: WO9324510

(56) Documents cités:
- EP-A- 0 217 580
- EP-A- 0 322 384
- EP-A- 0 481 214
- WO-A-91/14696
- FR-A- 2 654 106
- F.PUECH et al. Bioorg.Med.Chem.Letters 2, 603 (1992)
- G.GOSSELIN et al. Internat.Antiviral News 1, 100 (1993)

## Description

La présente invention concerne la fonctionnalisation bioréversible de groupes phosphates ou phosphonates de composés biologiquement actifs.

Plus particulièrement la présente invention concerne des composés biologiquement actifs du type phosphotriesters portant des groupes phosphates ou phosphonates protégés par des groupes protecteurs bioréversibles en milieu intracellulaire.

Les composés portant un groupe phosphate ou phosphonate ont une nature ionique chargée négativement et un pH physiologique. L'activité thérapeutique de tels composés est limitée de ce fait par la faible diffusion à travers les membranes biologiques lipidiques de composés négativement chargés. D'autre part, les composés portant des groupes phosphates sont sujets à une déphosphorylation aisée par l'action des enzymes phosphatases dans le sang ou sur les membranes cellulaires qui déphosphorylent les composés substrats. En général, les composés phosphates ou phosphonates chargés sont mal absorbés par administration orale et ne diffusent pas efficacement à travers les membranes cellulaires ou même la barrière cérébrale de nature lipidique.

Certains composés tels que les dérivés ou analogues de nucléosides sont actifs administrés sous forme non phosphorylée, mais sont phosphorylés in vivo sous forme de métabolique monophosphate ou triphosphate pour être actifs.

Ainsi les dérivés de nucléosides à activité antitumorale, tels que la 5-fluoro-uridine, la 5-fluoro-2'-désoxyuridine ou la 1-β-D-arabinofuranosylcytosine exercent leur activité sous forme phosphorylée.

De même, pour exercer leur activité antiproliférative, certains analogues de nucléosides ou de phosphononucléosides se doivent d'être phosphorylés par des enzymes cellulaires -ou virales- en leur triphosphate correspondant ; celui-ci étant ensuite susceptible d'inhiber les polymérases virales et/ou cellulaires.

Parmi les différentes classes structurales d'agents antiviraux, les 2',3' -didésoxynucléosides sont des composés qui présentent une des meilleure efficacité lors du traitement du SIDA. Mais ces analogues de nucléoside doivent subir une biotransformation par des kinases cellulaires pour exercer leur activité sur la réplication du VIH, l'agent étiologique du SIDA. Cette métabolisation se fait par passage par le didéoxynucléoside 5'-monophosphate puis le 5'-diphosphate pour conduire au 5'-triphosphate qui est un inhibiteur de la transcriptase inverse du VIH et qui interfère ainsi avec la biosynthèse de l'ADN viral.

Malgré leur grande potentialité thérapeutique, les 2',3'-didésoxynucléosides souffrent de limitations notamment la faible capacité de certains d'entre eux à être métabolisés par les kinases en triphosphate. La 2',3'-didésoxyuridine 5'-triphosphate par exemple, est un excellent inhibiteur de la transcriptase inverse (Z. Hao et coll., *Proc. Am., Assoc. Cancer Res.,* **1988**, 29, 348, E.Matthes et coll. , *Biochem. Biophys. Res. Commun,* **1987**, 148, 78-85). Par contre son nucléoside est inapte à inhiber la réplication du VIH in vitro. Des études ont montré que ce résultat est lié à la faible capacité du nucléoside à être métabolisé en son monophosphate par les kinases cellulaires (Z. Hao et coll. *Mol. Pharmacol.* **1990**, 37,157-153).

Ainsi, l'AZT est successivement métabolisée en son triphosphate (AZTP) qui est un puissant inhibiteur de la reverse transcriptase du VIH. De même, l'Acyclovir (ACV) est transformé en son triphosphate (ACVTP) qui inhibe sélectivement la DNA polymérase du virus de l'herpès. La première étape de l'activation des nucléosides (Nu) consiste en une monophosphorylation conduisant au monophosphate correspondant (NuMP). C'est cette première étape qui est la plus sélective.

Dans le but de passer outre cette étape clé de monophosphorylation enzymatique, on a déjà proposé d'administrer directement des NuMP, mais leur utilisation à des fins thérapeutiques s'est heurtée aux limitations et inconvénients mentionnés ci-après.

Les composés portant un groupe phosphate ou phosphonate ont une nature ionique chargée négativement à un pH physiologique. L'activité thérapeutique de tels composés est de ce fait limitée compte-tenu de la faible diffusion à travers les membranes biologiques lipidiques de composés négativement chargés. En particulier les composés chargés ne diffusent pas efficacement à travers les membranes cellulaires ni même à travers la barrière cérébrale de nature lipidique. D'autre part de tels composés sont sujets à une déphosphorylation aisée par l'action des enzymes phosphatases dans le sang ou sur les membranes cellulaires qui déphosphorylent les composés substrats de ces enzymes. En général les composés phosphates ou phosphonates chargés sont mal absorbés par administration orale.

On a cherché à transformer des mononucléotides en des phosphotriesters neutres susceptibles de traverser la membrane cellulaire et de délivrer intracellulairement le phosphotriester mononucléotidique (NuMP) correspondant. Un tel concept a été abordé par divers auteurs depuis de nombreuses années, mais s'est avéré décevant. Les dérivés obtenus présentaient en général soit une toxicité trop importante soit une stabilité extracellulaire insuffisante et n'apportaient finalement aucune amélioration au plan de l'activité biologique.

Ainsi l'utilisation de structures nucléosidiques phosphorylées comprenant des groupements protecteurs bioréversibles de type acyloxyméthyle ou acyloxybenzyle a été proposée pour des dérivés nucléosides anti-tumoraux tels ceux de la 5-fluorouracile dans les brevets WO n° 9008155 et WO 9119721. Toutefois ces composés ont une stabilité chimique limitée, et génèrent in vivo des métabolites formaldéhydes toxiques. Ils sont en outre peu solubles et leur rendement de préparation chimique est faible.

Le but de la présente invention est donc de fournir d'autres types de groupements bioréversibles pouvant être associés notamment à des structures mononucléotidiques ou autres de sorte que leur activité biologique s'en trouve augmentée, en particulier en ce qui concerne les composés dérivés ou analogues de nucléosides à activité anti-virale, et ne présentant pas les inconvénients précités.

La présente invention propose d'utiliser de nouveaux groupements caractérisés par la présence de liaisons enzymo-labiles -S 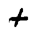 S- et/ou -S 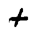 C=Z qui conduisent après activation enzymatique à la formation d'intermédiaires instables libérant sélectivement le monophosphate ou monophosphonate correspondant.

La présente invention a plus précisement pour objet le composé répondant à la formule générale I : 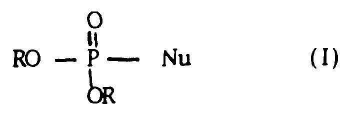 dans laquelle :
- R est un radical -(CH₂)ₙ-S-X
   où :
   - X représente un radical 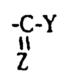 ou -S-U,
   - Z étant O ou S,
- Y et U représentant un radical alkyle, aryle ou osidique, éventuellement substitué notamment par un groupe OH, SH ou NH et,
   - n est égal à 1 à 4 de préférence 1 ou 2 et,
- Nu est un radical consistant en un reste de composé biologiquement actif ou le reste déphosphorylé d'un composé qui est biologiquement actif lorsq'il porte un groupe phosphate ou phosphonate.

Lorsque dans la formule (I) Nu est lié au phosphore par une liaison P-O, le composé de formule (I) selon l'invention porte un groupe phosphate et constitue donc un composé phosphotriester.

Lorsque Nu est lié au phosphore par une liaison P-C, le composé de formule (I) selon l'invention porte un groupe phosphonate.

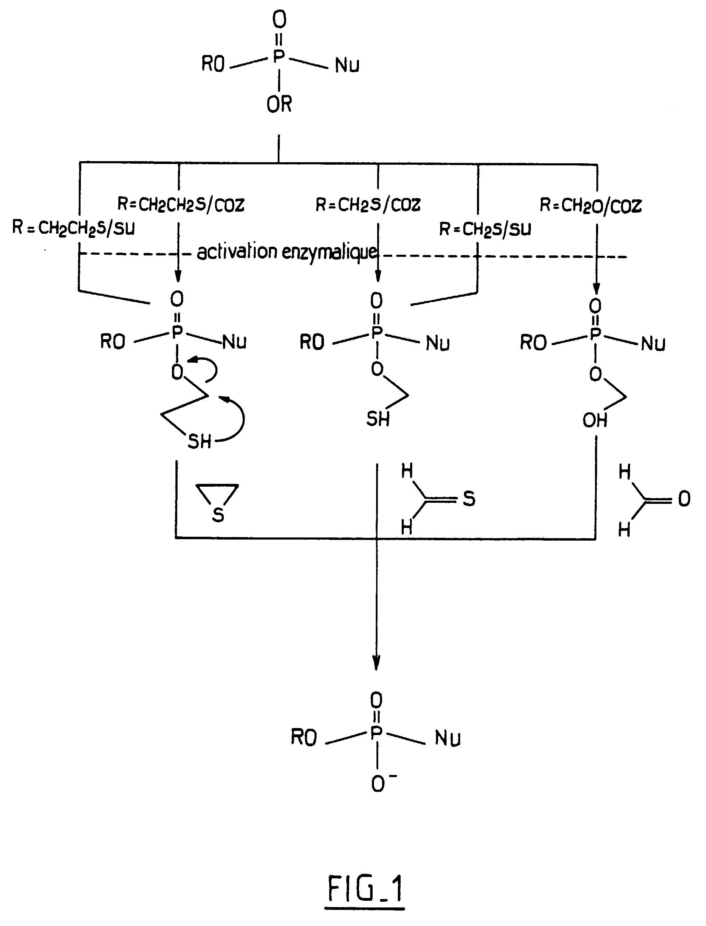

Les mécanismes de bioréversibilité des radicaux R se font par une coupure enzymatique des liaisons S-X et une libération des restes (CH₂)ₙ-S selon un mécanisme illustré par les exemples représentés figure 1.

On cite en particulier pour Y et U comme groupement alkyle, un alkyle en C₁ à C₇ ; comme groupement aryle, les radicaux phényle et benzyle et, comme radicaux osidiques, le glucose, le mannose ou le rhamnose.

Dans un mode de réalisation, lorsque X représente SU de préférence U représente le radical -(CH₂)ₙ¹ - X¹ où X₁ représente H, OH, SH, ou NH₂ et ₙ¹ est égal à 1 à 4, de préférence 1 ou 2.
On cite en particulier les composés (I) dans lesquels R représente -(CH₂)₂-S-S-(CH₂)₂-OH.

Dans un autre mode de réalisation lorsque X représente 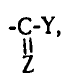 de façon appropriée Y représente CH₃ ou tBu.

On cite en particulier les composés (I) pour lesquels R représente 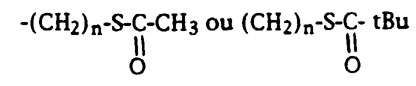 avec n = 1 ou 2.

Dans un mode de réalisation avantageux de la présente invention des composés (I), on cite en particulier les composés pour lesquels Nu représente un reste en 5' de nucléoside naturel ou de dérivé de nucléoside naturel thérapeutiquement actif ou dont le dérivé 5'-(O)-monophosphate ou 5'-(C)-monophosphonate est thérapeutiquement actif.

Ces composés de formule (I) ont en général une activité anti-virale ou anti-tumorale.

On cite plus particulièrement les composés de formule (I) pour lesquels Nu représente un reste en 5' de 2',3'- didéoxynucléoside ou 2',3'-didéhydronucléoside.

Parmi les composés (I) dérivés de didéoxynucléosides à activité antivirale on cite plus particulièrement les composés (I) pour lesquels Nu est un reste en 5' de la ddU (didéoxyuridine), la ddT (didéoxythymidine), la ddC (didéoxycytidine), l'AZT (3'-azido, 2',3'-didéoxythymidine) ainsi que leurs dérivés substitués notamment sur la base pyrimidique ou en 2' et 3' du cycle osidique.

La ddT, la ddC ou l'AZT illustrent les radicaux Nu qui représentent un reste en 5' de dérivé de nucléoside naturel thérapeutiquement actif.

La ddU illustre les radicaux Nu qui représentent un reste en 5' d'un dérivé de nucléoside qui n'est actif que sous forme phosphorylée. La ddU (didéoxyuridine) n'est pas monophosphorylée enzymatiquement in vivo. Seul son triphosphate est un inhibiteur de polymérase et lui confère une activité anti-virale.

Parmi les composés (I) à activité anti-tumorale on cite en particulier les composés pour lesquels Nu représente un reste en 5' des dérivés 5-fluoro-uridine ou 5-fluoro-2'-désoxyuridine ou 1-β-D-arabinofuranosylcytosine. Ces composés illustrent l'intérêt de la fonctionnalisation selon l'invention pour contourner la résistance acquise à certaines drogues nucléosidiques lorsque cette résistance est due à une perte de leur aptitude à être monophosphorylé comme c'est souvent le cas en chimiothérapie anti-tumorale.

Selon une autre variante de réalisation de l'invention dans les composés (I) le radical Nu représente un reste d'analogue de nucléoside tel qu'un carbonucléoside (nucléoside dont l'oxygène du cycle osidique est remplacé par un carbone), un phosphononucléoside (nucléoside dont l'oxygène en 5' est remplacé par un carbone) ou un dérivé de base purine ou pyrimidine du type acyclonucléoside c'est à dire ne comportant pas de cycle osidique tel que l'ACV (aciclovir) ou un radical méthoxyalkylpurine ou- pyrimidine de formule CH₂-O-alkyl -purine ou- pyrimidine.

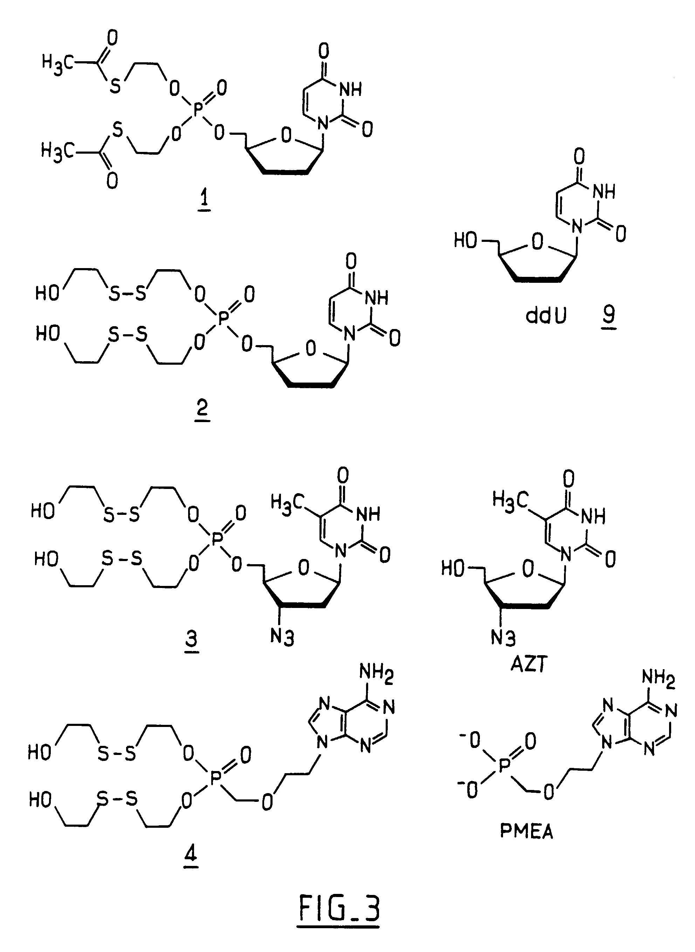

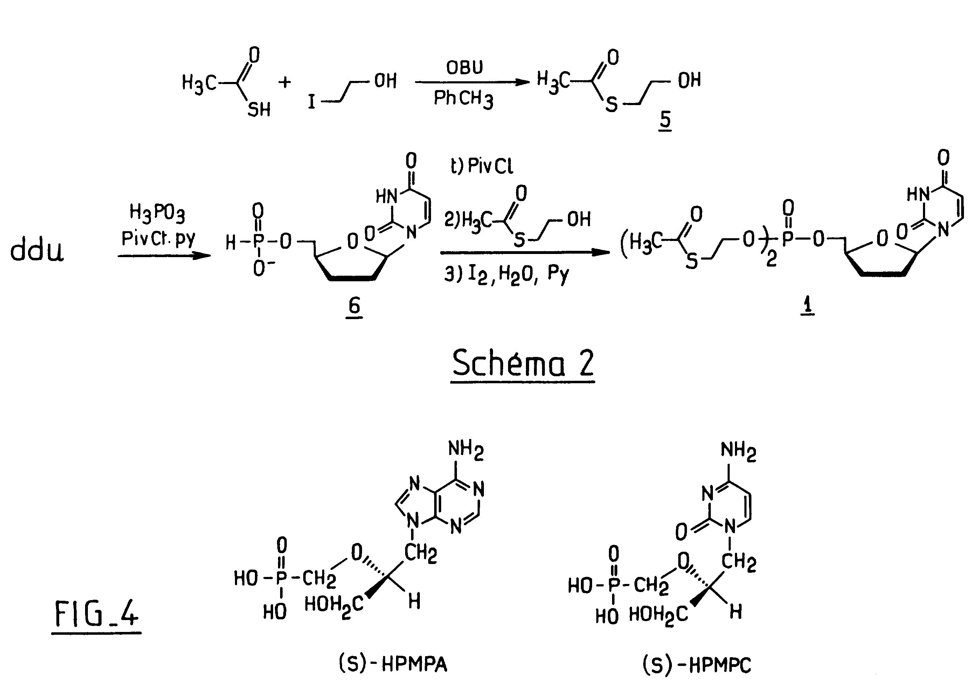

Les composés (I) pour lesquels Nu représente un radical méthoxyalkyl -purine ou- pyrimidine illustrent les composés phosphonates. Il s'agit en l'espèce de composés anti-viraux phosphonylméthoxyalkyl-purine ou- pyrimidine parmi lesquels on cite en particulier le PMEA, l'HPMPA ou l'HPMPC dont les formules sont données dans les figures 3 et 4.

La présente invention a donc en particulier pour objet des composés dans lesquels Nu est un radical 3-hydroxy-2-méthoxypropyl-purine ou-pyrimidine de formule : -CH₂-O-CH(CH₂OH)-CH₂. purine ou- pyrimidine ou un radical 2-méthoxyéthyl-purine ou- pyrimidine de formule -CH₂-O-C₂H₄ -purine ou- pyrimidine et par exemple les composés (I) pour lesquels Nu est un radical méthoxy-éthyl adénine ou 3-hydroxy -2-méthoxypropyl cytosine.

Lorsque Nu représente un reste déphosphorylé (déphosphaté ou déphosphonaté) d'une molécule qui est biologiquement active lorsqu'elle est sous forme phosphate ou phosphonate, la fonctionnalisation selon l'invention peut permettre d'une façon générale de moduler les paramètres physico-chimiques et biophysiques de ladite molécule comprenant un groupe phosphate ou phosphonate. Le composé (I) peut alors par exemple consister en un composé phosphopeptide ou phospholipide.

Lorsque Nu représente un reste de nucléoside, de dérivé ou d'analogue de nucléoside ceux ci peuvent être des énantiomères D ou L.

Les composés selon l'invention peuvent être préparés par des procédés connus de l'homme de l'art.

En particulier la présente invention a pour objet un procédé de préparation des composés selon l'invention caractérisé en ce que l'on prépare un composé de formule (I) dans lequel les groupes fonctionnels de R, et éventuellement de Nu, sont protégés par des groupes protecteurs appropriés puis l'on déprotège lesdits groupes fonctionnels de R, et éventuellement de Nu, pour obtenir les composés de formule (I).

En particulier de façon appropriée on fait réagir un composé de formule (II) : 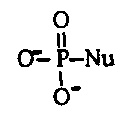 où Nu est éventuellement protégé, avec le composé de formule (III) X-S-(CH₂)ₙ-OH où X est protégé pour obtenir ledit composé de formule (I) protégé que l'on déprotège ensuite.

Dans un mode de réalisation particulier, la réaction entre les composés de formule (II) et (III) se fait en présence d'un agent de condensation tel que le MSNT dans de la pyridine.

D'autres procédés de préparation sont illustrés dans les exemples qui suivent dans lesquels apparaitront également d'autres caractéristiques et avantages de la présente invention.

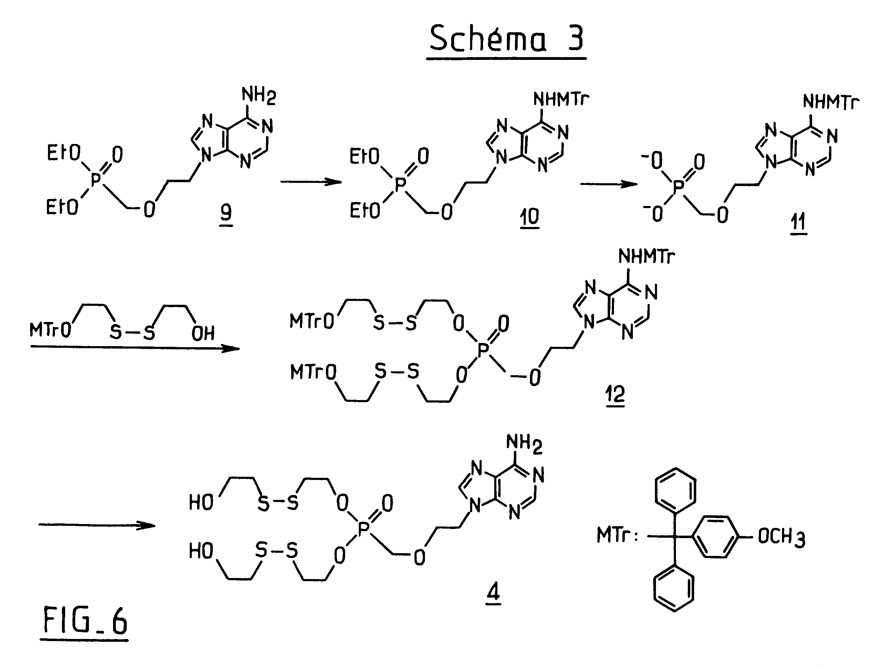

La description fait référence aux figures 1 à 6, dans lesquelles :
- **La figure 1** représente des mécanismes de décomposition des groupements bioréversibles sous activation enzymatique. Le même mécanisme se produit pour les deux groupes R.
- **La figure 2** représente le mécanisme de décomposition du groupe bioréversible du composé de l'exemple 2.
- **La figure 3** représente la formule de certains composés selon l'invention.
- **La figure 4** représente un schéma de préparation de composés préparés à l'exemple 1 et la formule des composés HPMPA et HPMPC.
- **La figure 5** représente les schémas de préparation de composés préparés aux exemples 2 et 3.
- **La figure 6** représente le schéma de préparation de composés préparés à l'exemple 4.

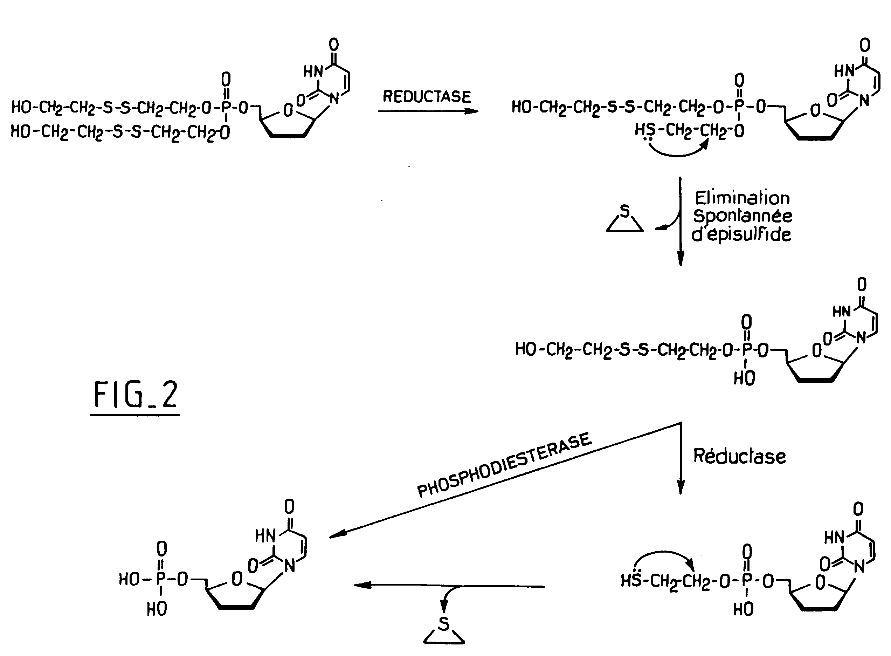

L'intérêt de cette invention résidant dans la différence de stabilité des phosphotriesters mononucléotidiques entre les milieux extra- et intra-cellulaires, il est tout d'abord montré que la décomposition de l'un des composés décrits dans l'invention (exemple 2) répond bien aux critères précédemment énnoncés et s'effectue selon le mécanisme présenté figure 2.

La technique HPLC "ISRP on line" *("On-fine InternaJ Surface Reversed-Phase Cleaning : The Direct HPLC Analysis of Crude Biological Samples",* A. POMPON, I. LEFEBVRE et J.L IMBACH, Biochemical Pharmacology, 43, 1769-1775 (1992) ) a été utilisé pour cette étude, le composé étudié étant respectivement incubé dans le milieu de culture (RPMI / 10% sérum inactivé) et dans un extrait cellulaire total (CEM).

Le composé de l'exemple 2 présente un temps de demi-vie de 9 heures en milieu de culture et de moins de 5 minute en extrait cellulaire. La libération intracellulaire de NuMP correspondant est corroborée par la mise en évidence d'une activité biologique, alors que le nucléoside constitutif est inactif.

De plus, dans la mesure où l'étape déterminant la vitesse d'activation du phosphotriester en mononucléotide est hautement dépendante de la cinétique d'hydrolyse enzymatique initiale, une variation de la nature des groupements enzymo-labiles conduit à une modulation des paramètres pharmacocinétiques de la drogue et aboutit à des effets retard.

Ces données valident clairement l'intérêt de l'invention.

Les chromatographies sur couche mince ont été réalisées sur plaques de silice Merck 60F 254 (art.5554). Les chromatographies sur colonne de gel de silice ont été effectuées avec de la silice Merck 60 H (art. 7736) ou avec de la silice silanisée RP2 Merck (art. 7719).
Avant analyse ou lyophilisation, les solutions ont été filtrées sur filtre Millex HV-4 (Millipore).

Les spectres UV ont été enregistrés sur un spectrophotomètre UVIKON 810.

Les spectres de masse ont été pris sur un appareil JEOL JMS DX 300 par la méthode d'ionisation FAB en mode positif ou négatif dans une matrice de glycérol (G), glycérol/thioglycérol (GT) ou d'alcool 3-nitrobenzylique (NBA).

Les spectres RMN du proton ont été enregistrés sur un appareil Varian EM 360 ou sur appareil Brüker AC 250.
Les déplacements chimiques sont exprimés en ppm par rapport au signal du tétraméthylsilane (TMS).
La multiplicité et l'allure des signaux observés par RMN sont indiquées par une (ou plusieurs) lettre(s) : s (singulet), d (doublet), t (triplet), m (multiplet), l (large).
Les spectres RMN du phosphore ont été enregistrés sur un appareil Brüker WP 200 SY avec découplage du proton.
Les déplacements chimiques sont exprimés en ppm par rapport au signal de H₃PO₄ pris comme référence externe.

### Exemple 1:

### O-(2',3'-didésoxyuridin-5'-yl) O,O'-bis(S-acétyl 2-thioéthyl)phosphate (1) (Schéma en figure 4)

### 2-Hydroxyéthyl acétyl sulfide (5)

Une solution de 1,0 ml (14 mmol.) d'acide thioacétique dans 5 ml de toluène est traitée avec 0,90 ml (12 mmol.) d'iodoéthanol en présence de 1,7 ml (12 mmol.) de 1,8-diazabicyclo-(5,4,0) undéc-7-ène (DBU) pendant 2 heures. Le milieu réactionnel est dilué avec du dichlorométhane et lavé avec de l'eau. La phase organique est séchée sur sulfate de sodium et évaporée. Le brut obtenu est purifié sur colonne de gel de silice (èluant : méthanol (0-4%) dans le dichlorométhane) pour conduire à 1,2 g (85%) de **5** sous forme d'huile.
**5** : RMN¹H (DMSO-*d*_{*6*}): d = 2,32 (s, 3H, CH₃): 2,91 (t, 2H, CH₂S, J = 6,6 Hz): 3,45 (pseudo q, 2H, CH₂OH, J = 6 Hz); 4,97 (t, 1H, OH) ppm.

### O-(2',3'-didésoxyuridin-5'-yl)-hydrogénophosphonate (6)

Une solution 1,5 M d'acide phosphoreux (165 ml, 247 mmol.) dans la pyridine anhydre est ajoutée à 5,25 g de 2',3'-didésoxyuridine (24,7 mmol.) et est traitée avec 16,8 ml de chlorure de pivaloyle (136 mmol.). Après 3 heures de réaction, une solution aqueuse 1 M de bicarbonate de triéthylammonium est ajoutée jusqu'à neutralisation et le solvant est évaporé sous pression réduite. L'huile obtenue est chromatographiée sur colonne de gel de silice (éluant : méthanol (0-35%) dans le dichlorométhane) pour conduire à **6**. Le produit est repris dans du méthanol et est filtré sur filtre Millipore. L'évaporation du solvant donne 7,10 g (76%) de **6** (forme triéthylammonium) suffisamment pur pour la suite de la synthèse. Un échantillon de plus grande pureté est obtenu après une purification supplémentaire par chromatographie sur couche mince de gel de silice utilisant un mélange d'isopropanol, ammoniaque, eau (8:1:1:) comme éluant. Le produit sous forme ammonium est extrait de la silice avec du méthanol, le solvant est chassé par évaporation et le résidu est repris à l'eau, filtré sur filtre Millipore et lyophilisé.
**6**: UV(H₂O) : 1max = 262 nm (e 9940): 1min = 230 nm (e 2080)
SM (FAB négatif, GT) : 275 (M)⁻
RMN¹H (DMSO-*d*_{*6*}) : d = 1,78-2,05 (m, 3H, H-2',3',3"); 2,18-2,45 (m, 1H, H-2"); 3,65-3,95 (m, 2H, H-5',5"); 4,11 (m, 1H, H-4'); 5,55 (d, 1H, H-5, J = 8,1 Hz); 5,95 (dd, 1H, H-1'; J = 6,8 et 3,8 Hz); 6,63 (d, 1H, HP, J = 592 Hz); 7,87 (d, 1H, H-6, J = 8,1 Hz) ppm
RMN³¹P (DMSO-*d*_{*6*}) : d = 1,60 ppm.

### O-(2',3'-didésoxyuridin-5'-yl) O,O'-bis(S-acétyl 2-thioéthyl) phosphate (1)

Une solution de 200 mg (0,530 mmol.) de l'hydrogénophosphonate **6** de la 2',3'-didésoxyuridine dans 5 ml de pyridine est traitée avec 196 µl de chlorure de pivaloyle pendant 30 minutes. 159 mg (1,33 mmol.) de 2-hydroxyéthyl acétyl sulfide (**5**) sont ajoutés et la réaction est laissée 2 heures sous agitation. Le phosphite formé est oxydé à l'aide d'une solution d'iode à 2% dans un mélange pyridine-eau (98:2) jusqu'à coloration persistante (7-8 ml). Le solvant est évaporé sous pression réduite. Le brut obtenu est coévaporé avec du toluène et chromatographié sur colonne de gel de silice (éluant : méthanol (0-6%) dans le dichlorométhane) pour conduire à 65 mg (25%) du composé 1 sous forme d'huile.
**1**: UV (EtOH) : 1max = 262 nm (e 9400); 1min = 230 nm (e 2500)
SM (FAB positif) : 497 (M+H)⁺
RMN¹H (DMSO-*d6*) : d = 1,73-2,13 (m, 3H, H-2',3',3"); 2,20-2,4 (m, 1H, H-2"); 2,356 et 2,360 (s et s, 3H et 3H, 2 CH₃); 3,13 (t, 4H, 2 CH₂S, J = 6,4 Hz); 4,00-4,26 (m, 7H, H-4', 5',5" et 2 CH₂CH₂OP); 5,60 (d, 1H, H-5, J = 8,1 Hz); 6,01 (dd, 1H, H-1', J = 4,2 et 7,0 Hz); 7,64 (d, 1H, H-6, J = 8,1 Hz); 11,3 (s1, 1H, NHCO) ppm.
RMN³¹P (DMSO-*d*_{*6*}) : d = -1,21 ppm.

### Exemple 2:

### O,O'-Bis (S-(2-hydroxyéthylsufidyl) 2-thioéthyl) O-(2',3'-didésoxyuridin-5'-yl)phosphate (2).

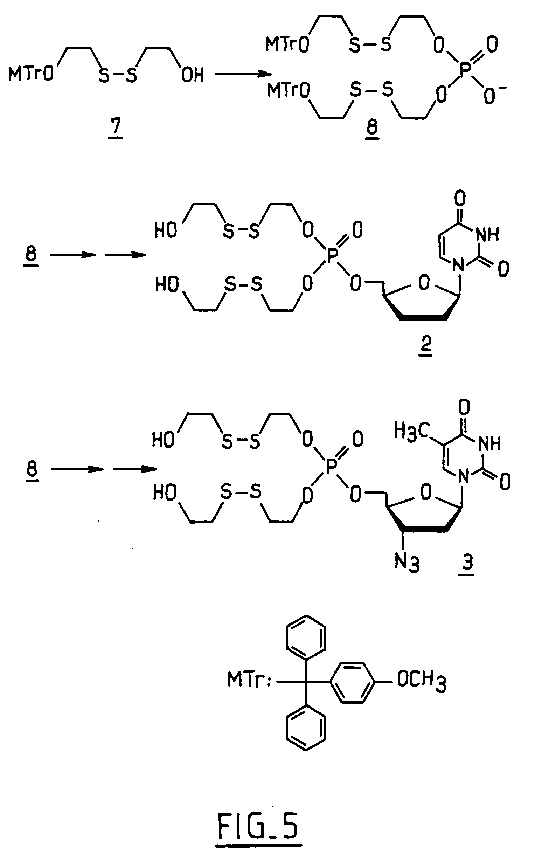

### (Schéma en figure 5)

### O,O'-Bis (S-(O-(4-méthoxytrityl) 2-oxéthylsufidyl 2-thioéthyl) phosphate (8).

A une solution de 0,910 g (13,4 mmoles) d'imidazole dans 18 ml de pyridine à 0°C sont ajoutés 0,406 ml (4,45 mmoles) d'oxychlorure de phosphore. Le mélange est agité 30 minutes à température ambiante puis ajouté à 3,80 g (8,91 mmoles) de mono-O (4-méthoxymtyl) dithiodiéthanol (7). Après 18 heures, le mélange réactionnel est traité avec une solution 1 M d'acétate de triéthylammonium. Les produits de la réaction sont extrait avec du dichlorométhane et la phase organique est lavée avec de l'eau, séchée sur sulfate de sodium, concentrée sous pression réduite et coévaporée avec du toluène. Une purification sur colonne de gel de silice (éluant : méthanol (0-10%) dans le dichlorométhane) conduit à 2,2 g (48%) de **8** sous forme de sel de triéthylammonium.
**8**: SM (FAB négatif, NBA) : 913 (M⁻).
RMN¹H (DMSO-*d6*) : 1,14 (t, 9H, (CH₃CH₂)₃NH⁺, J = 7,3 Hz); 2.78 (t, 4H, 2 SCH₂CH₂OP, J = 6,4 Hz). 2,86 (t, 4H, 2 SCH₂CH₂OMTr, J = 6 Hz); 2,99 (q, 6H, (CH₃CH₂)₃NH⁺, J = 7,3 Hz); 3,21 (t, 4H, 2 CH₂OMTr, J = 5,9 Hz): 3,71 (s, 6H, 2 CH₃O); 3,87 (m, 4H, 2 CH₂OP); 6,82-7,45 (m, 28H, 2 Tr) ppm.
RMN³¹P (DMSO-*d6*) : - 2.70 ppm.

### O,O'-Bis (S-(2-hydroxyéthylsufidyl) 2-thioéthyl) O-(2',3'-didésoxyuridin-5'-yl) phosphate (2).

Un mélange de 666 mg (0,655 mmoles) de **8** et de 139 mg (0.656 mmoles) de 2',3'-didésoxyuridine dans 5 ml de pyridine est traité avec 486 mg (1,64 mmoles) de 1-(2-mésitylènesulfonyl) 3-nitro 1,2,4-triazole. Après 30 heures, le mélange réactionnel est dilué avec du dichlorométhane, lavé avec une solution aqueuse 1M d'acétate de triéthylammonium, puis avec de l'eau. La phase organique est séchée sur sulfate de sodium, concentrée sous pression réduite, coévaporée avec du toluène et chromatographiée sur colonne de gel de silice (éluant : méthanol (0-4%) dans le dichlorométhane). Le phosphotriester protégé partiellement purifié est traité avec 5 ml du mélange acide acétique/eau/méthanol (8:1:1) pendant 24 heures. Les solvants sont chassés par évaporation sous pression réduite et l'huile obtenue est coévaporée avec du toluène. Une purification sur colonne de gel de silice (éluant : méthanol (0-6%) dans le dichlorométhane) suivie d'une purification sur colonne de silice silanisée (éluant : éthanol (0-40%) dans l'eau) conduit à 52 mg (14%) du composé **2** après lyophilisation dans le dioxanne.
- **2** : UV (EtOH) :: λ max 261 nm (ε 9900)
λ min 231 nm (ε 3100).
SM (FAB positif, GT) : 565 (M+H)⁺: 489 (M-SCH₂CH₂OH+2H)⁺: 429 (M-HOCH₂CH₂SSCH₂CH₂+2H)⁺.
RMN¹H (DMSO-*d6*) : 1,63-1,9 (m, 1H, H-3'); 1,9-2,10 (m, 2H, H-2',3"); 2,33-2,40 (m, 1H, H-2"); 2,80 (t, 2H, HOCH₂CH₂S, J = 6,4 Hz); 2,81 (t, 2H, HOCH₂CH₂S, J = 6,4 Hz); 3,00 (t, 4H, 2 SCH₂CH₂OP, J = 6,3 Hz); 3,61 (pseudo q, 4H, 2 HOCH₂, J = 6 Hz), 4,07-4,32 (m, 7H, H-4',5',5" et 2 CH₂CH₂OP); 4,89 (t, 2H, 2 HO, J = 4,9 Hz); 5,598 (d, 1H, H-5, J = 8,1 Hz); 5,604 (d, 1H, H-5, J = 8,1 Hz); 6,00 (dd, 2H, 2 H-1', J = 4,1 et 7,9 Hz); 7,65 (d, 2H, 2 H-6, J = 8,0 Hz); 11,31 (s1, 1 H, NHCO) ppm.
RMN³¹P (DMSO-*d6*) : - 0,880 ppm.

### Exemple 3:

### O,O'-Bis (S-(2- hydroxyéthylsufidyl) 2-thioéthyl O-(3'-azido 3'-désoxythymidin-5'-yl) phosphate (3).

### (Schéma en figure 5)

Un mélange de 666 mg (0,655 mmoles) de **8** et de 193 mg (0,722 mmoles) de 3'-azido 3'-désoxythymidine dans 5 ml de pyridine est traité avec 486 mg ( 1,64 mmoles) de 1-(2-mésitylènesulfonyl) 3-nitro 1,2,4-triazole. Après 24 heures, 194 mg (0,656 mmoles) de 1-(2-mésitylènesulfonyl) 3-nitro 1,2,4 triazole sont rajoutés et la réaction est laissée 24 heures supplémentaires. Le mélange réactionnel est alors dilué avec du dichlorométhane, lavé avec une solution aqueuse 1M d'acétate de triéthylammonium, puis avec de l'eau. La phase organique est séchée sur sulfate de sodium, concentrée sous pression réduite, coévaporée avec du toluène et chromatographiée sur colonne de gel de silice (éluant : méthanol (0-2%) dans le dichlorométhane). Le phosphotriester protégé partiellement purifié est traité avec 5 ml du mélange acide acétique/eau/méthanol (8:1:1) pendant 24 heures. Les solvants sont chassés par évaporation sous pression réduite et l'huile obtenue est coévaporée avec du toluène. Une purification sur colonne de gel de silice (éluant : méthanol (0-6%) dans le dichlorométhane) conduit à 130 mg 129%) du composé **3** après lyophilisation dans le dioxanne.
- **3** : UV(EtOH):: λ max 264 nm (ε 9600)
λ min 234 nm (ε 2100).
SM (FAB positif, GT) : 620 (M+H)⁺; 544 (M-SCH₂CH₂OH+2H)⁺.
RMN¹H (DMSO-*d6*) : 1,80 (s, 3H, CH₃); 2,26-2,5 (m, 2H,H-2',2"); 2,796 (t, 2H, HOCH₂CH₂S, J = 6,4 Hz); 2,802 (t, 2H, HOCH₂CH₂S, J = 6,4 Hz); 2,99 (t, 4H, 2 SCH₂CH₂OP, J = 6,3 Hz); 3,61 (pseudo q, 4H, 2 HOCH₂, J = 6 Hz); 4,02 (m, 1H, H-4'); 4,09-4,44 (m, 6H, H-5',5" et 2 CH₂CH₂OP); 4,48 (m, 1H, H-3'); 4,90 (t, 2H, 2 HO, J = 5,3 Hz); 6,14 (t, 1H, H-1', J = 6,6 Hz); 7,49 (s, 1H, H-6); 11,37 (s1, 1H, NHCO) ppm.
RMN³¹P (DMSO-*d6*) : - 0,954 ppm.

### Exemple 4:

### 9-(2-(O,O'-Bis(S(2-hydroxyéthylsufidyl)-2-thioéthyl)phosphonylméthoxy-éthyl)adénine (4).

### (Schéma en figure 5)

### N⁶-(4-Méthoxytrityl)9-(2-diéthoxyphosphonylméthoxyéthyl)adénine (10).

Une solution de 3,93 g (11,9 mmoles) de 9-(diethoxyphosphonylméthoxyéthyl) adénine (**9**) (A. Holy et coll., *Collection Czechoslovak Chem. Commun. 52,* 2792, **1987**) et de 146 mg (1,19 mmoles) de 4-diméthylaminopyridine dans 50 ml de dichlorométhane est traitée avec 3,31 ml (23,8 mmoles) de triéthylamine et 7,35 g (23,8 mmoles) de chlorure de 4-méthoxytrityle pendant 4 heures. Le mélange réactionnel est ensuite dilué avec du dichlorométhane et lavé avec une solution aqueuse d'hydrogénocarbonate de sodium puis avec de l'eau. La phase organique est séchée sur sulfate de sodium et concentrée sous pression réduite. Une chromatographie sur colonne de gel de silice (éluant : méthanol (0-3%) dans dichlorométhane) permet d'isoler 5.43 g (84%) du composé **10**.
- **10** : UV (EtOH) :: λ max 275 nm (ε 27200)
λ min 246 nm (ε 11200).
SM (FAB négatif, GT) : 601 (M-H)⁻; 406 (A^{MTr})⁻; 328 (M-MT^{r})⁻
RMN¹H (DMSO-*d6*) : 1,10 (t, 6H, 2 CH₃CH₂, J = 7,0 Hz); 3,71 (s, 3H, CH₃O), 3,80-3,98 (m, 4H, PCH₂ et CH₂CH₂); 3,88 (q, 4H, 2 CH₃CH₂, J = 8 Hz); 4,33 (t, CH₂CH₂, J = 4,8 Hz); 6,80-7,37 (m, 14H, Tr); 7,91 (s, 1H, H-8): 8,18 (s, H-2) ppm.
RMN³¹P (DMSO-*d6*) : 21,35 ppm.

### N⁶-(4-Méthoxytrityl) 9-(2-phosphonylméthoxyéthyl)adénine (11).

Une solution de 5,00 g (8,31 mmoles) de **10** dans 29 ml d'acétonitrile est traitée avec 3,29 ml (24,9 mmoles) de bromure de triméthylsilyle pendant 14 heures. L'excès de réactif et le solvant sont chassés par évaporation sous pression réduite. L'huile obtenue est reprise avec du bicarbonate de triéthylammonium et concentré sous pression réduite. La purification est réalisée par chromatographie sur colonne de gel de silice (éluant : méthanol (0-50%) dans le dichlorométhane). Après filtration en solution dans du dichlorométhane, 3,4 g (63%) de **11** sont isolés sous forme de sel mixte d'acide et de triéthylammonium (1:1).
**11** : SM (FAB négatif, GT) : 544 (M-H)⁻: 272 (M-MTr)⁻.
RMN¹H (DMSO-*d6*) : 1,11 (t, 9H, (CH₃CH₂)NH⁺, J = 7,3 Hz); 2,96 (q, 6H, (CH₃CH₂)NH⁺, J = 7,3 Hz); 3,34 (d, 2H, PCH₂, J = 8,4 Hz); 3,68 (s, 3H, CH₃O); 3,8 (m, 2H, CH₂CH₂); 4,27 (t, CH₂CH₂, J = 4,5 Hz); 6,65-7,35 (m, 14H, Tr); 7,83 (s, 1H, H-8); 8,31 (s, 1H, H-2) ppm.
RMN³¹P (DMSO-*d6*) : 11,40 ppm.

### N⁶-(4-Méthoxytrityl)9-(2-(O,O'-Bis(S-(O-(4-méthoxytrityl)2-oxéthylsufidyl) 2-thioéthyl)) phosphonylméthoxyéthyl) adénine (12).

Un mélange de 296 mg (0,458 mmoles) de **11** avec 977 mg (2,29 mmoles) de mono-O (4-méthoxytrityl) dithiodiéthanol (**7**) dans 5 ml de pyridine est traité avec 341 mg (1,15 mmoles) de 1-(2,mésitylènesulfonyl) 3-nitro 1,2,4 triazole. Après 3 jours, le mélange réactionnel est dilué avec du dichlorométhane, lavé avec une solution aqueuse saturée d'hydrogénocarbonate de sodium, puis avec de l'eau. La phase organique est séchée sur sulfate de sodium, concentrée sous pression réduite, coévaporée avec du toluène et chromatographiée sur colonne de gel de silice (éluant : méthanol (0-5%) dans le dichlorométhane) pour donner 330 mg (53%) de **12**.
- **12** : UV (EtOH) :: λ max 275 nm (ε 28200)
λ min 253 nm (ε 18300).
SM (FAB négatif, NBA) : 1360 (M-H)⁻; 952 (M-MTrOCH₂CH₂SSCH₂CH₂)⁻. RMN¹H (DMSO-*d6*) : 2,75 (t, 4H, 2 SCH₂CH₂OP, J = 6,3 Hz), 2,86 (t, 4H, 2 SCH₂CH₂OMTr, J = 5,9 Hz); 3,19 (t, 4H, 2 CH₂OMTr, J = 6,0 Hz); 3,68 (s, 3H, CH₃O); 3,69 (s, 6H, 2 CH₃O); 3,83 (m, 4H, PCH₂ et CH₂CH₂); 4,05 (m, 4H, 2 CH₂OP); 4,28 (t, 2H, CH₂CH₂, J = 4,6 Hz); 6,87-7,45 (m, 42H, 3 Tr); 7,88 (s, 1H, H-8); 8,12 (s, 1H, H-2) ppm.
RMN³¹P (DMSO-*d6*) : 22,09 ppm.

### 9-(2-(O,O'-Bis(S-(2-hydroxyéthylsufidyl)2-thioéthyl)phosphonylméthoxyéthyl) adénine (4).

Le phosphotriester **12** (290 mg, 0.213 mmoles) est traité avec 15 ml du mélange acide acétique/eau/méthanol (8:1:1) pendant 15 heures. Les solvants sont chassés par évaporation sous pression réduite et l'huile obtenue est coévaporée avec du toluène. Une purification sur colonne de gel de silice (éluant : méthanol (0-8%) dans le dichlorométhane) conduit à 116 mg (90%) du composé **4** après lyophilisation dans le mélange eau/dioxanne.
- **4** : UV (EtOH):: λ max 260 nm (ε 14700)
λ min 228 nm (ε 3600).
SM (FAB positif, GT) : 545 (M+H)⁺.
RMN¹H (DMSO-*d6*) : 2,80 (t, 4H, 2 SCH₂CH₂OP, J = 6,4 Hz); 2,91 (t, 4H, 2 SCH₂CH₂OH, J = 6,4 Hz); 3,61 (pseudo q, 4H, 2 CH₂OH, J = 6 Hz); 3,91 (t, 2H, CH₂CH₂, J = 5,1 Hz); 3,95 (d, 2H, PCH₂, J = 8,2 Hz); 4,15 (m, 4H, 2 CH₂OP); 4,32 (t, 2H, CH₂CH₂, J = 5,0 Hz); 7,20 (s1, 2H, NH₂); 8,08 (s, 1H, H-8); 8,14 (s, 1H, H-2) ppm.
RMN³¹P (DMSO-*d6*) : 22,24 ppm.

### EXEMPLE 5 :

### EVALUATION DE L'ACTIVITE ANTI-VIH 1 SUR LES CELLULES CEM ET MT-4

VIH = virus de l'immunodéficience humaine
MT-4 = cellule leucémique T humaine
CEM = cellule lymphoblastoïde T humaine

La réplication du VIH-1 (isolat LAI) dans les cellules CEM est mesurée par un dosage de la réverse transcriptase (RTase) dans le surnageant de culture après 5 jours d'infection. Cette activité traduit la présence du virus libéré par les cellules. Après l'adsorption du virus, les composés testés sont ajoutés à différentes concentrations dans le milieu de culture.

L'activité antivirale est exprimée par la concentration la plus faible de composé qui diminue la production de RTase d'au moins 50% (ED₅₀).

L'effet toxique sur les CEM non infectées est apprécié par une réaction colorimétrique basée sur la capacité des cellules vivantes à réduire le bromure de 3-(4,5 diméthylthiazol-2-yl)-2,5 diphényltétrazolium en formazan après 5 jours d'incubation en présence de différentes concentrations des composés. Les résultats sont exprimés par la concentration la plus faible de composé qui provoque une inhibition d'au moins 50% de la formation de formazan (CD₅₀).

Les composés exemplifiés dans cette invention présentent les activités anti-HIV suivantes :
. *Composé 1* :
   - ED₅₀ -: CEM-TK⁻, 4.10⁻⁶ M (CD₅₀ 7.10⁻⁵ M)
   CEM-SS, 5.10⁻⁶ M (CD₅₀ 9.10⁻⁵ M)
   MT4 , 2.10⁻⁶ (CD₅₀ 9.10⁻⁵ M)
. *Composé 2 :*
   - ED₅₀ -: CEM-TK⁻, 8.10⁻⁶ M (CD₅₀ 8.10⁻⁵ M)
   CEM-SS, 6.10⁻⁵ M (CD₅₀ 10⁻⁴ M)
. *Composé 3 :*
   - ED₅₀ -: CEM-TK⁻, 7.10⁻⁶ M (CD₅₀ 8.10⁻⁵ M)
   CEM-SS, 7.10⁻¹⁰ M (CD₅₀ 8.10⁻⁵ M)
   MT4 , 10⁻⁹ M (CD₅₀ 8.10⁻⁵ M)
. *Composé 4 :*
   - ED₅₀ -: CEM-TK⁻, 8.10⁻⁸ M (CD₅₀ : 4.10⁻⁵ M)
   CEM-SS, 3.10⁻⁶ M (CD₅₀ > 10⁻⁴ M)
   MT4 , 8.10⁻⁷M (CC₅₀ : 2.10⁻⁵ M)

L'ensemble de ces données confirme bien qu'il y a eu libération intracellulaire du monophosphate nucléosidique.

## Revendications

1. Composé phosphotriester répondant à la formule générale I : 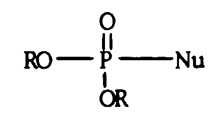 dans laquelle :
- R est un radical -(CH₂)ₙ-S-X
où :
- X représente un radical 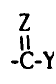 ou -S-U,
- Z étant O ou S,
- Y et U représentant un radical alkyle, aryle ou osidique, éventuellement substitués notamment par un groupe OH, SH ou NH₂ et,
- n est égal à 1 à 4 de préférence 1 ou 2 et,
- Nu est un radical consistant en un reste d'un composé biologiquement actif ou le reste déphosphorylé d'un composé qui est biologiquement actif lorsq'il porte un groupe phosphate ou phosphonate.

2. Composé selon la revendication 1 caractérisé en ce que X représente -S-U et, U représente le radical (CH₂)ₙ¹-X¹ où X¹ représente H, OH, SH ou NH₂ et n¹ est égal à 1 à 4 de préférence 1 ou 2.

3. Composé selon la revendication 2 caractérisé en ce que R représente -(CH₂)₂-S-S-(CH₂)₂ OH.

4. Composé selon la revendication 1 caractérisé en ce que X représente 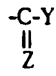 et représente CH₃ ou tBu.

5. Composé selon la revendication 4 caractérisé en ce que R représente 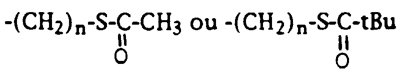 avec n=1 ou 2.

6. Composé selon l'une des revendications 1 à 5 caractérisé en ce que Nu représente un reste en 5' de nucléoside naturel, ou de dérivé de nucléoside naturel thérapeutiquement actif ou dont le dérivé 5'-(O) monophosphate ou 5' -(C) monophosphonate est thérapeutiquement actif.

7. Composé selon la revendication 6 caractérisé en ce que Nu représente un reste en 5' d'un dérivé de nucléoside naturel du type 2', 3'-didéoxynucléoside ou 2', 3'-didéhydronucléoside.

8. Composé selon la revendication 7 caractérisé en ce que Nu représente un reste en 5' de la ddU, ddT, ddC, de l'AZT, ou un dérivé de ces derniers substitué sur la base pyrimidique notamment en position 5 de celle-ci ou en 2' et 3' du cycle osidique.

9. Composé selon l'une des revendications 1 à 5 caractérisé en ce que Nu représente un reste d'analogue de nucléoside tel qu'un carbonucléoside, un phosphononucléoside ou un acyclonucléoside.

10. Composé selon la revendication 9 caractérisé en ce que Nu est un radical du type méthoxyalkyl -purine ou- pyrimidine de formule -CH₂-O-alkyl purine ou- pyrimidine.

11. Composé selon la revendication 10 caractérisé en ce que Nu est un radical 3-hydroxy-2-méthoxypropyl-purine ou pyrimidine de formule -CH₂-O-CH(CH₂OH)-CH₂-purine ou- pyrimidine ou, un radical 2-méthoxyéthylpurine ou- pyrimidine de formule -CH₂-O-C₂H₄-purine ou- pyrimidine.

12. Procédé de préparation d'un composé selon l'une des revendications 1 à 11 caractérisé en ce que l'on prépare un composé de formule (I) dans lequel les groupes fonctionnels de R, et éventuellement de Nu, sont protégés par des groupes protecteurs appropriés puis l'on déprotège lesdits groupes fonctionnels de R, et éventuellement de Nu, pour obtenir les composés de formule (I).

13. Procédé selon la revendication 12 caractérisé en ce que l'on fait réagir un composé de formule (II) : 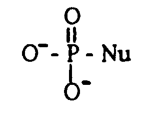 où Nu est éventuellement protégé, avec le composé de formule (III) :
X-S-(CH₂)ₙ-OH
où X est protégé pour obtenir ledit composé de formule (I) protégé que l'on déprotège ensuite.

14. Procédé selon la revendication 13 caractérisé en ce que la réaction entre les composés de formules (II) et (III) se fait en présence d'un agent de condensation tel que le MSNT dans de la pyridine.

## Patentansprüche

1. Phosphotriester-Verbindung der allgemeinen Formel (I): 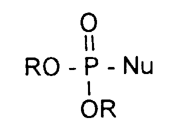 worin bedeuten:
R einen Rest -(CH₂)ₙ-S-X, worin darstellen:
- X einen Rest 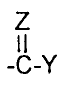 oder -S-U,
- Z O oder S,
- Y und U einen Alkyl-, Aryl- oder Osid-Rest, die gegebenenfalls insbesondere durch eine OH-, SH- oder NH₂-Gruppe substituiert sind, und
- n eine Zahl von 1 bis 4, vorzugsweise die Zahl 1 oder 2, und
Nu einen Rest, der besteht aus einem Rest einer biologisch aktiven Verbindung oder dem dephosphorylierten Rest einer Verbindung, die biologisch aktiv ist, wenn sie eine Phosphat- oder Phosphonat-Gruppe trägt.

2. Verbindung nach Anspruch 1, dadurch gekennzeichnet, daß X steht für -S-U und U steht für den Rest (CH₂)ₙ¹-X¹, worin X¹ H, OH, SH oder NH₂ darstellt und n¹ eine Zahl von 1 bis 4, vorzugsweise die Zahl 1 oder 2, ist.

3. Verbindung nach Anspruch 2, dadurch gekennzeichnet, daß R steht für -(CH₂)₂-S-S-(CH₂)₂OH.

4. Verbindung nach Anspruch 1, dadurch gekennzeichnet, daß X steht für 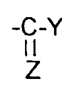 und Y steht für CH₃ oder tBu.

5. Verbindung nach Anspruch 4, dadurch gekennzeichnet, daß R steht für 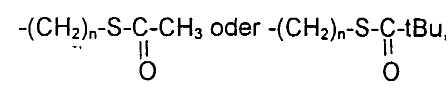 worin n = 1 oder 2.

6. Verbindung nach einem der Ansprüche 1 bis 5, dadurch gekennzeichnet, daß Nu steht für einen Rest in 5'-Stellung eines natürlichen Nucleosids oder eines Derivats eines natürlichen Nucleosids, das therapeutisch aktiv ist oder dessen 5'- (O) -Monophosphat- oder 5'-(C)-Monophosphonat-Derivat therapeutisch aktiv ist.

7. Verbindung nach Anspruch 6, dadurch gekennzeichnet, daß Nu steht für einen Rest in 5'-Stellung eines natürlichen Nucleosid-Derivats vom 2', 3'-Dideoxynucleosid- oder 2', 3'-Didehydronucleosid-Typ.

8. Verbindung nach Anspruch 7, dadurch gekennzeichnet, daß Nu steht für einen Rest in 5'-Stellung von ddU, ddT, ddC, AZT oder ein Derivat dieser letztgenannten, das an der Pyrimidin-Base insbesondere in 5-Stellung desselben oder in 2'- oder 3'-Stellung des Osid-Ringes substituiert ist.

9. Verbindung nach einem der Ansprüche 1 bis 5, dadurch gekennzeichnet, daß Nu steht für einen Rest eines Nucleosid-Analogons wie eines Carbonucleosids, eines Phosphononucleosids oder eines Acyclonucleosids.

10. Verbindung nach Anspruch 9, dadurch gekennzeichnet, daß Nu steht für einen Rest vom Methoxyalkyl-purin- oder -pyrimidin-Typ der Formel -CH₂-O-alkyl-purin oder -pyrimidin.

11. Verbindung nach Anspruch 10, dadurch gekennzeichnet, daß Nu steht für einen 3-Hydroxy-2-methoxypropyl-purin oder -pyrimidin-Rest der Formel -CH₂-O-CH(CH₂OH)-CH₂-Purin oder -Pyrimidin oder einen 2-Methoxyethylpurin- oder -pyrimidin-Rest der Formel -CH₂-O-C₂H₄-Purin oder -Pyrimidin.

12. Verfahren zur Herstellung einer Verbindung nach einem der Ansprüche 1 bis 11, dadurch gekennzeichnet, daß man eine Verbindung der Formel (I) herstellt, in der die funktionellen Gruppen von R und gegebenenfalls von Nu durch geeignete Schutzgruppen geschützt werden und daß man dann die genannten funktionellen Gruppen von R und gegebenenfalls von Nu von den Schutzgruppen befreit unter Bildung der Verbindungen der Formel (I).

13. Verfahren nach Anspruch 12, dadurch gekennzeichnet, daß man eine Verbindung der Formel (II) 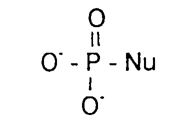 worin Nu gegebenenfalls geschützt ist, mit der Verbindung der Formel (III):
X-S-(CH₂)ₙ-OH
worin X geschützt ist, reagieren läßt unter Bildung der genannten geschützten Verbindung der Formel (I), die man anschließend von den Schutzgruppen befreit.

14. Verfahren nach Anspruch 13, dadurch gekennzeichnet, daß die Umsetzung zwischen den Verbindungen der Formeln (II) und (III) in Gegenwart eines Kondensationsmittels wie MSNT in Pyridin durchgeführt wird.

## Claims

1. Phosphotriester compound corresponding to the general formula I: 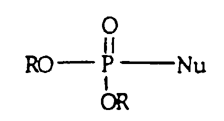 in which:
- R is a radical -(CH₂)ₙ-S-X
where:
- X represents a radical 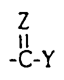 or -S-U,
- Z being O or S,
- Y and U representing an alkyl, aryl or saccharide radical which are optionally substituted, in particular with an OH, SH or NH₂ group, and
- n is equal to 1 to 4, preferably 1 or 2, and
- Nu is a radical consisting of a residue of a biologically active compound or the dephosphorylated residue of a compound which is biologically active when it bears a phosphate or phosphonate group.

2. Compound according to Claim 1, characterized in that X represents -S-U and U represents the radical (CH₂)ₙ¹-X¹ where X¹ represents H, OH, SH or NH₂ and n¹ is equal to 1 to 4, preferably 1 or 2.

3. Compound according to Claim 2, characterized in that R represents -(CH₂)₂-S-S-(CH₂)₂OH.

4. Compound according to Claim 1, characterized in that that X represents 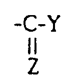 and Y represents CH₃ or tBu.

5. Compound according to Claim 4, characterized in that R represents 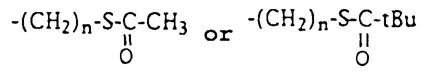 with n = 1 or 2.

6. Compound according to one of Claims 1 to 5, characterized in that Nu represents a 5' residue of a natural nucleoside or of a derivative of a natural nucleoside, which is therapeutically active or for which the 5'-(O)-monophosphate or 5'-(C)-monophosphonate derivative is therapeutically active.

7. Compound according to Claim 6, characterized in that Nu represents a 5' residue of a natural nucleoside derivative of 2',3'-dideoxynucleoside type or 2',3'-didehydronucleoside type.

8. Compound according to Claim 7, characterized in that Nu represents a 5' residue of ddU, ddT, ddC, AZT or a derivative thereof which is substituted on the pyrimidine base, especially in position 5 thereof, or at 2' and 3' of the saccharide ring.

9. Compound according to one of Claims 1 to 5, characterized in that Nu represents a nucleoside analogue residue such as a carbonucleoside, a phosphononucleoside or an acyclonucleoside.

10. Compound according to Claim 9, characterized in that Nu is a radical of methoxyalkylpurine or -pyrimidine type, of formula -CH₂-O-alkylpurine or -pyrimidine.

11. Compound according to Claim 10, characterized in that Nu is a 3-hydroxy-2-methoxypropylpurine or -pyrimidine radical, of formula -CH₂-O-CH(CH₂OH)-CH₂-purine or -pyrimidine, or a 2-methoxyethylpurine or -pyrimidine radical, of formula -CH₂-O-C₂H₄-purine or -pyrimidine.

12. Process for the preparation of a compound according to one of Claims 1 to 11, characterized in that a compound of formula (I) is prepared, in which compound the functional groups of R, and optionally of Nu, are protected by suitable protecting groups, followed by deprotection of the said functional groups of R, and optionally of Nu, in order to obtain the compounds of formula (I).

13. Process according to Claim 12, characterized in that a compound of formula (II) : 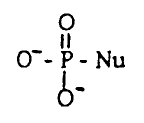 where Nu is optionally protected, is reacted with the compound of formula (III):
X-S-(CH₂)ₙ-OH
where X is protected, in order to obtain the said protected compound of formula (I), which is then deprotected.

14. Process according to Claim 13, characterized in that the reaction between the compounds of formulae (II) and (III) takes place in the presence of a condensing agent such as MSNT, in pyridine.
